# EUROPÄISCHE PATENTSCHRIFT

(11) **EP 0 588 267 B1**
(45) Veröffentlichungstag und Bekanntmachung des Hinweises auf die Patenterteilung: **29.12.1999**
(21) Anmeldenummer: 93114614.6
(22) Anmeldetag: 10.09.1993
(51) Int. Cl.: C13J 1/00, C13J 1/06, C13D 3/14

(54) **Verfahren zur Weiterverarbeitung von technischen Restabläufen der melasseverarbeitenden Industrie**
Method for further processing technical refuse from molasses industry
Procédé de traitement ultérieur de résidus techniques de l'industrie de la mélasse

(30) Priorität: 17.09.1992 DE 4231149
(43) Veröffentlichungstag der Anmeldung: 23.03.1994
(73) Patentinhaber: AMINO GmbH, D-38373 Frellstedt (DE)
(72) Erfinder: Smolnik, Heinz-Dieter. Dipl.-Ing, 38126 Braunschweig (DE); Thommel, Jürgen. Dipl.-Ing, 38304 Wolfenbüttel (DE)
(74) Vertreter: Einsel, Martin, Dipl.-Phys.

(56) Entgegenhaltungen:
- EP-A- 0 345 511
- WO-A-81/02420
- US-A- 2 785 179
- US-A- 3 884 714

## Beschreibung

Die Erfindung betrifft ein Verfahren zur Weiterverarbeitung von technischen Restabläufen der Melasseverarbeitung, beispielsweise von Vinasse (Schlempe) oder Restmelasse mittels Ionenausschlußchromatographie.

Bei der Melasseverarbeitung fällt als technischer Restablauf insbesondere Vinasse bzw. Schlempe oder Restmelasse an. Diese Produkte sind bisher nur schwer zu verwerten und auch ihre Entsorgung bereitet Schwierigkeiten.

Eine erste Aufgabe der Erfindung besteht daher darin, ein Verfahren zur Weiterverarbeitung vorzuschlagen, mit dem die zu entsorgenden Mengen verringert und/oder noch wirtschaftlich verwertbare Inhaltsstoffe gewonnen werden können.

Als wichtiger Inhaltsstoff derartiger technischer Restabläufe käme etwa Betain in Betracht.

Betain ((CH₃)₃-N⁺-CH₂-COO⁻, C₅H₁₁NO₂) kommt in verschiedenen Pflanzenarten, vor allem aber in der Zuckerrübe, vor. Bei der Zuckergewinnung aus Zuckerrüben reichert sich das Betain in der Melasse an; die Konzentration liegt im Bereich von 3 bis 8 % Betain in der Trockensubstanz.

Auch bei vielen biotechnologischen Prozessen mit Melasse als Rohstoff (Kohlenstoffquelle), wie z.B. bei der Hefe- oder Alkoholproduktion wird Betain nicht assimiliert und reichert sich deshalb in den Endabläufen dieser Verfahren an, etwa in der Vinasse (Melasseschlempe). Die Betaingehalte können dabei auf über 15 % der Trockensubstanz ansteigen.

Ähnliche Gehalte werden auch in den Restmelassen bei Melasseentzuckerungsverfahren, z.B. nach dem Baryt- oder Steffen-Verfahren, gefunden.

Betain wird hauptsächlich wegen seiner Eigenschaft als Methylgruppen-Donator als Zusatzstoff für Futtermittel verwendet. Daneben wird Betain aber auch in kosmetischen und pharmazeutischen Präparaten eingesetzt, etwa als Hautpflegemittel bzw. Leberschutzpräparat.

Für die Gewinnung von Betain sind verschiedene Verfahren entwickelt worden. Dazu gehören die Gewinnung durch Kristallisation als Hydrochlorid, durch Extraktion mit Hilfe organischer Lösungsmittel, durch Ionenaustausch und insbesondere mittels Ionenausschlußchromatographie, wie dies beispielsweise in der EP 0 054 544 B1 vorgeschlagen wird.

Dieses in der Praxis vielfach erprobte Verfahren zur Gewinnung von Betain aus Melasse läuft wie folgt ab: Ein Kationenaustauscher wird mit der aufzubereitenden Lösung beschickt. Dabei wird die Melasse so verdünnt, daß sie einen Feststoffgehalt im ungefähren Bereich von 20 bis 50 % besitzt. Im Kationenaustauscher finden im wesentlichen zwei Effekte gleichzeitig nebeneinander statt, der Ionenausschluß und die Molekülsiebwirkung. Die starken Elektrolyte wandern relativ ungestört durch die Trennkolonne, während die schwachen Elektrolyte und Nichtelektrolyte vom Austauscher adsorbiert werden. Durch die Elution mit Wasser werden die adsorbierten Stoffe wieder freigesetzt. Dabei werden wegen der unterschiedlichen Wanderungsgeschwindigkeiten durch den Austauscher die Elektrolyte und Nichtelektrolyte voneinander getrennt, und man erhält Fraktionen mit unterschiedlicher Zusammensetzung.

Während die verdünnte Melasse als einheitliche Charge auf die Oberseite einer Trennkolonne gegeben wird, erscheinen nach Durchlaufen der Trennkolonne die verschiedenen Substanzen in einer zeitlich getrennten Reihenfolge. Da die Bestandteile der Ursprungssubstanz im wesentlichen konstant und bekannt sind, kann auch recht genau vorhergesagt werden, wann welche Bestandteile im Auslauf der Trennkolonne erscheinen, so daß die einzelnen Fraktionen gezielt voneinander getrennt werden können.

Die Fraktionen der verschiedenen Chargen sind zwar gleichbleibend zusammengesetzt, können jedoch in bestimmten Grenzen durch gezielte Wahl der Prozeßparameter in geeigneter Weise optimiert werden.

Eine ganz exakte Trennung der Inhaltsstoffe ist allerdings nicht möglich; zwischen den einzelnen Fraktionen gibt es jeweils gewisse Mischmengen.

Die bisherigen Verfahren gehen davon aus, daß Abläufe der Zuckerrübenverarbeitung, wie z.B. Melasse, durch mehrfache Trennung nach dem Ionenauschlußverfahren mit Wasser als Elutionsmittel aufgereinigt werden. Der Einsatz von Wasser sorgt für eine möglichst saubere Trennung der einzelnen Fraktionen voneinander und hält die "Mischabläufe" relativ klein. Bei der Verwendung anderer Elutionsmittel würden auch zusätzliche Fremdstoffe eingeführt werden, so daß davon bisher abgesehen wurde.

Für die bekannten Verfahren, wie sie z. B. in der EP 0 054 544 B1, der WO 81/02420, der US-PS 3,884,714 und der DE 22 32 093 C3 beschrieben sind, wird zwar vorgeschlagen, produktarme Zwischenfraktionen in einem semikontinuierlichen Chargenbetieb zur Ausbeuteverbesserung zurückzuführen, wobei sie zudem als Eluierungsmittel dienen können, jedoch erfolgt die Eluierung im ersten Trennungsgang und zwischen den einzelnen Chargen immer mit Wasser.

Trotz der an sich guten Ergebnisse des bekannten Verfahrens ist nachteilig, daß hohe Eindampf- und Aufbereitungskosten entstehen, da am Ausgang der Trennkolonne die einzelnen Fraktionen große Wassermengen enthalten. Dies führt dazu, daß die Gewinnung der wertvollen Inhaltsstoffe, insbesondere des Betains, aus technischen Restabläufen nicht immer wirtschaftlich ist. Dies gilt insbesondere für die Verarbeitung von Vinasse oder Restmelasse, wo nicht wie bei der Zuckergewinnung aus Melasse ein weiterer Kostenträger zur Verfügung steht.

Eine weitere Aufgabe der Erfindung ist es daher, das gattungsgemäße Verfahren so weiterzuentwickeln, daß eine kostengünstigere Gewinnung von Betain auch aus solchen Restabläufen möglich wird.

Die genannten Aufgaben werden dadurch gelöst, daß mindestens im ersten Trennungsgang durch die Trennkolonne die Elution der zu trennenden Stoffe mit dünner oder rückverdünnter Lösung des Ausgangsstoffes erfolgt.

Die Maßnahme erscheint im Hinblick auf die angestrebte Absicht zunächst widersinnig. Durch die Verwendung einer Lösung des Ausgangsproduktes zur Elution werden die an sich in der Trennkolonne erzielten und angestrebten deutlichen Abstände zwischen den verschiedenen Fraktionen "verschmiert", sie können also weniger deutlich voneinander unterschieden werden. Durch die Elution mit gerade den gleichen Materialien, wie sie ohnehin voneinander getrennt werden sollen, wird nämlich deren Erscheinen im Auslauf zeitlich jeweils etwas gestreckt, so daß die Überlappungsbereiche deutlich zunehmen.

Der Vorteil dieser zunächst widersinnig erscheinenden Maßnahme besteht jedoch darin, daß alle im Auslauf erscheinenden Fraktionen jeweils auch erheblich angereichert werden. Insbesondere nimmt der Wassergehalt der erscheinenden Fraktionen deutlich ab, so daß sich auch die nachfolgenden Eindampfungs- und Aufbereitungskosten entsprechend verringern.

Durch die entstehende höhere Konzentration einer jeden Fraktion wird es möglich, bei ihrer in der zeitlichen Abfolge geschehenden Zuordnung, die durch das Verschmieren etwas fraglicher werdenden Randbereiche einzugrenzen, also an sich auf Teile des gewonnenen Betains zu verzichten. Da in den verbleibenden Bereichen jedoch jetzt der Betainanteil im Verhältnis zur Trockensubstanz angestiegen ist, wird gleichwohl eine höhere Reinheit sichergestellt.

Eine Durchführung von mehreren Trennungsgängen nacheinander der gleichen Substanz mußte auch im Stand der Technik durchgeführt werden, um eine sichere Trennung zu gewährleisten und die entstehenden Produkte nach bekannten Methoden weiter aufarbeiten zu können.

In der Praxis besonders bewährt hat es sich, wenn die Elution mit dünner oder rückverdünnter Lösung der Ausgangssubstanz mit einer Konzentration zwischen 2 und 12 % Trockensubstanz, vorzugsweise mit 5 %, durchgeführt wird.

Bei diesen Konzentrationen hat sich eine besonders gute Abstimmung der einzelnen Effekte aufeinander herausgestellt, d.h., die "Verschmierung" der einzelnen Fraktionen bleibt noch relativ gering, andererseits findet bereits eine erhebliche Erhöhung der Konzentrationen generell statt.

Bevorzugt findet der erste und ggf. ein zweiter Trennungsgang mit einer Elution der Ausgangssubstanz statt.

Danach empfiehlt es sich, noch einen oder mehrere Trennungsgänge mit Wasser durchzuführen, die jetzt jedoch mit einer erheblich höheren Ausgangsreinheit stattfinden.

Vinasse und andere Restabläufe der Melasseverarbeitung enthalten noch weitere wertvolle Inhaltstoffe, beispielsweise Pyrrolidoncarbonsäure (Glutaminsäure). Diese Bestandteile von Vinasse konnten bisher kaum wirtschaftlich verwertbar gewonnen werden. Dabei ist in der nachveröffentlichten europäischen Patentanmeldung 91 104 968.2 ein Verfahren zur Herstellung eines Feuchthaltemittels vorgeschlagen worden, bei dem gerade diese Inhaltsstoffe eine wesentliche Rolle spielen.

Eine weitere Aufgabe der Erfindung ist es daher, ein Verfahren zur Gewinnung von Aminosäuren und anderen organischen Säuren aus technischen Restabläufen der Melasseverarbeitung vorzuschlagen.

Auch diese Aufgabe wird dadurch gelöst, daß mindestens im ersten Trennungsgang durch eine Trennkolonne die Elution der zu trennenden Stoffe mit dünner oder rückverdünnter Lösung des Ausgangsstoffes erfolgt.

Das Gewinnen dieser weiteren, wertvollen Inhaltsstoffe kann sogar zugleich mit dem Verfahren zur Gewinnung von Betain durchgeführt werden. Diese Inhaltsstoffe werden nämlich bei dem Ionenausschlußverfahren in einem vom Betain-Schwerpunkt getrennten Zeitbereich die Trennkolonne verlassen.

Die bei den Trennungsgängen anfallenden Fraktionen können dann gezielt weiter aufbereitet werden. So fällt nicht nur Betain, das eigentliche Hauptprodukt an, sondern auch γ-Aminobuttersäure (GABA), L-Asparaginsäure, Glycin, L-Alanin, L- Leucin, L-Serin, L-Valin, L-Isoleucin, L-Threonin, Milchsäure u.a. Außerdem ist in der Vinasse vor allem L-Glutaminsäure enthalten, die in Form ihres Umwandlungsproduktes als L-Pyrrolidoncarbonsäure (PCS) vorliegt. Sie entsteht unter Abspaltung von Ammoniak durch Zyklisierung aus L-Glutamin.

Als Kationenaustauscher in den Trennkolonnen sind Polystyrolharze mit einer Divinylbenzolvernetzung von nominal 3 bis 7 % gut geeignet. Die aktiven Gruppen sind Sulfonsäuregruppen, vorzugsweise in Natriumform. Die Korngröße der Harze liegt zwischen 0,2 und 0,5 mm, wobei vor allem eine gleichmäßige Größenverteilung von großer Bedeutung für die Trennqualität ist.

Durch das erfindungsgemäße Verfahren mit der Aufkonzentration der wertgebenden Fraktionen werden Lösungen mit hoher Reinheit erhalten, die entweder direkt als konzentrierte Lösung vermarktet werden können oder zur Gewinnung von Aminosäuren und anderen organischen Säuren sowie Betain durch Trocknung bzw. fraktionierte Kristallisation verwendet werden können.

Im folgenden soll die Durchführung der Erfindung noch anhand eines Ausführungsbeispieles näher erörtert werden, zu dem in der Zeichnung zusätzliche Verdeutlichung gegeben wird.

Es zeigen:
- Fig. 1: den zeitlichen Ablauf am Ausgang der Trennkolonne; die Zeit ist nach rechts und der Anteil Trockensubstanz TS nach oben aufgetragen und
- Fig. 2: eine schematische Darstellung des Verfahrensablaufs.

Eine doppelwandige Trennkolonne 1 mit einem Durchmesser von 0,1 m wird mit 30 l Kationenaustauscherharz in Natriumform gefüllt. Über den Doppelmantel wird die Temperatur auf ca. 85°C gebracht. Die Harzkügelchen werden mit einer 5 %igen Vinasselösung gesättigt und bis zur Gleichgewichtseinstellung von dieser 5 %igen Vinasselösung durchströmt. Dann werden 4 l einer konzentrierten Vinasselösung (bei 11) von ca. 35 % TS (Aufgabelösung) auf die Harzoberfläche aufgegeben. Die Aufgabelösung wird anschließend mit 5 %iger Vinasselösung (bei 12) eluiert. Hierbei muß sichergestellt sein, daß die Vinasselösungen vor der Verwendung in geeigneter Weise von Trüb- und Schwebstoffen gereinigt worden sind.

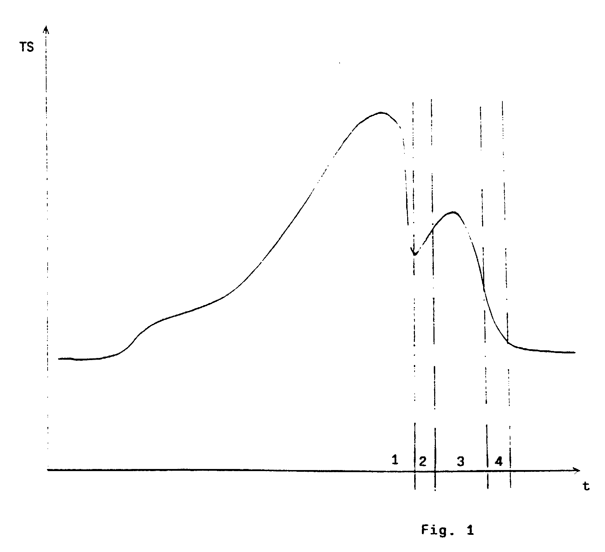

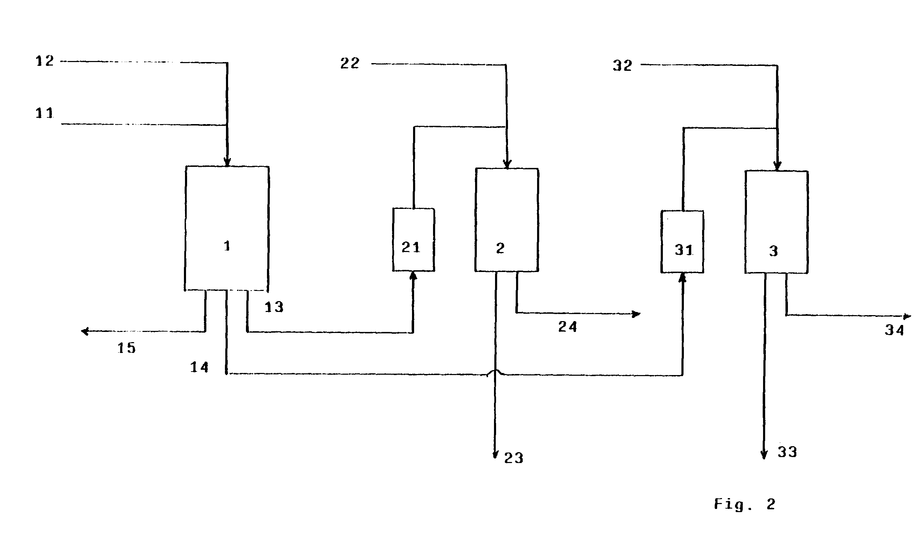

Die aus der Trennkolonne 1 austretenden Lösungen werden kontinuierlich über ein Differentialrefraktometer und ein Leitfähigkeitsmeßgerät kontrolliert, die Werte aufgezeichnet. Über einen Mikroprozessor können aufgrund der ermittelten Dichte- und Leitfähigkeitswerte sowie der Laufzeit als Parameter folgende Fraktionen voneinander getrennt werden (**Fig. 1**);
1. Salz- und Aminosäurefraktion (L-Pyrrolidoncarbonsäure), wird zur separaten Aufarbeitung abgeleitet (bei 14 in Fig. 2).
2. Rückführung 1 wird zur Aufgabelösung zurückgenommen (bei 15 in Fig. 2).
3. Betain-Fraktion (bei 13 in Fig. 2)
4. Rückführung 2, wird zur Elutionslösung zurückgeführt (bei 15 in Fig. 2).

Die Betain-Fraktion (bei 13) wird zur weiteren Aufarbeitung gesammelt. Sie enthält im Durchschnitt etwa 40 % Betain in der Trockenmasse. Die Betainlösung wird dann auf ca. 35 % Trockenmasse eingedampft (bei 21) und auf einer weiteren Trennkolonne 2 mit Wasser (Kondensat, bei 22) als Elutionsmittel in einem zweiten oder mehrfachen Trennungsgang weiter aufgereinigt. Das Verfahren entspricht dem oben bereits beschriebenen Trennprozeß. Die Reinheit dieser gereinigten Betain-Fraktion beträgt ca. 90 % in der Trockenmasse. Diese Betain-Fraktion (bei 23) kann deshalb entweder gleich direkt bzw. nach Eindampfung als konzentrierte Lösung vermarktet werden, oder aber das Betain wird nach bekannten Verfahren kristallisiert und in hoher Reinheit und Ausbeute gewonnen. Es verbleibt (bei 24) eine Abwasserfraktion.

Auch die anfallende (bei 14) Salz- und Aminosäurefraktion bzw. Fraktion organischer Säuren, die auch die L-Pyrrolidoncarbonsäure enthält, wird nach Eindampfung (bei 31) auf einer weiteren Trennkolonne 3 mit Wasser (Kondensat, bei 32) als Elutionsmittel in einem zweiten oder mehrfachen Trennungsgang weiter aufgereinigt. Die aufkonzentrierten organischen Säuren können jetzt der nachfolgenden Aufarbeitung (bei 33) zugeführt werden; bei 34 fällt noch eine Abwasserfraktion an. Nach Ablauf eines Trennungsganges kann sofort der nächste Zyklus begonnen werden.

## Patentansprüche

1. Verfahren zur Weiterverarbeitung von technischen Restabläufen der Melasseverarbeitung, beispielsweise von Vinasse (Schlempe) oder Restmelasse, mittels Ionenausschlußchromatographie,
**dadurch gekennzeichnet,**
daß mindestens im ersten Trennungsgang durch eine Trennkolonne die Elution der zu trennenden Stoffe mit dünner oder rückverdünnter Lösung des Ausgangsstoffes erfolgt.

2. Verfahren zur Gewinnung von Betain aus technischen Restabläufen der Melasseverarbeitung, beispielsweise aus Vinasse (Schlempe) oder Restmelasse, mittels Ionenausschlußchromatographie,
**dadurch gekennzeichnet,**
daß mindestens im ersten Trennungsgang durch die Trennkolonne die Elution der zu trennenden Stoffe mit dünner oder rückverdünnter Lösung des Ausgangsstoffes erfolgt.

3. Verfahren zur Gewinnung von Aminosäuren und anderen organischen Säuren, insbesondere Pyrrolidoncarbonsäure (Glutaminsäure) aus technischen Restabläufen der Melasseverarbeitung, beispielsweise aus Vinasse (Schlempe) oder Restmelasse, mittels Ionenausschlußchromatographie,
**dadurch gekennzeichnet,**
daß mindestens im ersten Trennungsgang durch eine Trennkolonne die Elution der zu trennenden Stoffe mit dünner oder rückverdünnter Lösung des Ausgangsstoffes erfolgt.

4. Verfahren nach einem der vorstehenden Ansprüche,
**dadurch gekennzeichnet,**
daß die Konzentration der dünnen bzw. rückverdünnten Lösung des Ausgangsstoffes mit einer Konzentration zwischen 2 und 12 % Trockensubstanz durchgeführt wird, vorzugsweise mit einer Konzentration von 5 % Trockensubstanz.

5. Verfahren nach Anspruch 2 oder 4,
**dadurch gekennzeichent,**
daß die nach dem ersten Trennungsgang gewonnene Betainfraktion konzentriert und -falls erforderlich- in einem zweiten Trennungsgang erneut mit dünner bzw. rückverdünnter Lösung des Ausgangsstoffes eluiert wird.

6. Verfahren nach Anspruch 2 oder 4 oder 5,
**dadurch gekennzeichnet,**
daß die gewonnene Betainfraktion konzentriert und nach den Trennungsgängen mit der Ausgangslösung noch mit einem oder mehreren Trennungsgängen mit Wasser eluiert und angereichert wird.

7. Verfahren nach einem der Ansprüche 3 bis 6,
**dadurch gekennzeichnet,**
daß die nach dem ersten Trennungsgang gewonnene Aminosäurefraktion konzentriert und -falls erforderlich- in einem zweiten Trennungsgang erneut mit dünner bzw. rückverdünnter Lösung des Ausgangsstoffes eluiert wird.

8. Verfahren nach einem der Ansprüche 3 bis 7,
**dadurch gekennzeichnet,**
daß die gewonnene Aminosäurefraktion konzentriert und nach den Trennungsgängen mit der Ausgangslösung noch mit einem oder mehreren Trennungsgängen mit Wasser eluiert und angereichert wird

9. Verfahren nach einem der vorstehenden Ansprüche,
**dadurch gekennzeichnet,**
daß vor der Einführung in die Trennkolonne die Vinasse bzw. Restmelasse durch Entfernung von Kalzium- und Magnesiumsalzen vorbehandelt wird.

10. Verfahren nach einem der vorstehenden Ansprüche,
**dadurch gekennzeichnet,**
daß während des Trennvorganges die Temperatur im Bereich von 70 bis 95°C gehalten wird.

## Claims

1. Method for the further processing of industrial residue discharges from molasses processing, for example of vinasse (slop) or residual molasses, by means of ion-exclusion chromatography,
characterised in that
at least in the first separation pass through a separating column the elution of the substances to be separated takes place with dilute or back-diluted solution of the starting material.

2. Method for the obtaining of betaine from industrial residue discharges from molasses processing, for example of vinasse (slop) or residual molasses, by means of ion-exclusion chromatography,
characterised in that
at least in the first separation pass through the separating column the elution of the substances to be separated takes place with dilute or back-diluted solution of the starting material.

3. Method for the obtaining of amino acids and other organic acids, in particular pyrrolidone carboxylic acid (glutamic acid), from industrial residue discharges from molasses processing, for example of vinasse (slop) or residual molasses, by means of ion-exclusion chromatography,
characterised in that
at least in the first separation pass through a separating column the elution of the substances to be separated takes place with dilute or back-diluted solution of the starting material.

4. Method according to any one of the preceding claims,
characterised in that
the concentrating of the dilute or back-diluted solution of the starting material is carried out with a concentration between 2 and 12% dry matter, preferably with a concentration of 5% dry matter.

5. Method according to claim 2 or 4,
characterised in that
the betaine fraction obtained after the first separation pass
is concentrated and - if necessary - eluted again in a second separation pass with dilute or back-diluted solution of the starting material.

6. Method according to claim 2 or 4 and 5,
characterised in that
the betaine fraction obtained is concentrated and after the separation passes with the starting solution also eluted and enriched with one or more separation passes with water.

7. Method according to any one of claims 3 to 6,
characterised in that
the amino acid fraction obtained after the first separation pass is concentrated and - if necessary - eluted again in a second separation pass with dilute or back-diluted solution of the starting material.

8. Method according to any one of claims 3 to 7,
characterised in that
the amino acid fraction obtained is concentrated and after the separation passes with the starting solution also eluted and enriched with one or more separation passes with water.

9. Method according to any one of the preceding claims,
characterised in that
prior to introduction into the separating column the vinasse or residual molasses is pre-treated by the removal of calcium and magnesium salts.

10. Method according to any one of the preceding claims,
characterised in that
during the separation pass the temperature is held in the range from 70 to 95°C.

## Revendications

1. Procédé de traitement ultérieur de résidus techniques du traitement de la mélasse, par exemple de vinasse (résidu de la distillation) ou de mélasse résiduelle, au moyen d'une chromatographie par exclusion ionique,
caractérisé en ce que, au moins lors du premier procédé de séparation par un fractionnateur, l'élution des substances à séparer est réalisée avec une solution de la matière de départ diluée ou reconstituée par dilution.

2. Procédé d'obtention de bétaïne à partir de résidus techniques du traitement de la mélasse, par exemple à partir de vinasse (résidu de la distillation) ou de mélasse résiduelle, au moyen d'une chromatographie par exclusion ionique,
caractérisé en ce que, au moins lors du premier procédé de séparation par un fractionnateur, l'élution des substances à séparer est réalisée avec une solution de la matière de départ diluée ou reconstituée par dilution.

3. Procédé d'obtention d'aminoacides et d'autres acides organiques, particulièrement d'acide carboxylique de pyrrolidone (acide glutamique) à partir de résidus techniques du traitement de la mélasse, par exemple à partir de vinasse (résidu de la distillation) ou de mélasse résiduelle, au moyen d'une chromatographie par exclusion ionique,
caractérisé en ce que, au moins lors du premier procédé de séparation par un fractionnateur, l'élution des substances à séparer est réalisée avec une solution de la matière de départ diluée ou reconstituée par dilution.

4. Procédé selon l'une des revendications précédentes,
caractérisé en ce que la concentration de la solution diluée ou reconstituée par dilution de la matière de départ est réalisée avec une concentration comprise entre 2 et 12 % de substance sèche, de préférence avec une concentration égale à 5 % de substance sèche.

5. Procédé selon la revendication 2 ou 4,
caractérisé en ce que la fraction de bétaïne obtenue après le premier procédé de séparation est concentrée et - si nécessaire - à nouveau éluée lors d'un second procédé de séparation avec une solution diluée ou reconstituée par dilution de la matière de départ.

6. Procédé selon la revendication 2 ou 4 ou 5,
caractérisé en ce que la fraction de bétaïne obtenue est concentrée et, après les procédés de séparation avec la solution de départ, encore éluée avec de l'eau par un ou plusieurs procédés de séparation, et concentrée.

7. Procédé selon l'une des revendications 3 à 6,
caractérisé en ce que la fraction d'aminoacide obtenue après le premier procédé de séparation est concentrée et - si nécessaire - à nouveau éluée lors d'un second procédé de séparation avec une solution diluée ou reconstituée par dilution de la matière de départ.

8. Procédé selon l'une des revendications 3 à 7,
caractérisé en ce que la fraction d'aminoacides obtenue est concentrée et, après les procédés de séparation avec la solution de départ, éluée avec de l'eau par un ou plusieurs procédés de séparation, et concentrée.

9. Procédé selon l'une des revendications précédentes,
caractérisé en ce que, avant introduction dans le fractionnateur, la vinasse ou la mélasse résiduelle est pré-traitée par élimination des sels de calcium et de magnésium.

10. Procédé selon l'une des revendications précédentes,
caractérisé en ce que, lors du procédé de séparation, la température est maintenue dans une gamme allant de 70 à 95 °C.
